# EUROPEAN PATENT SPECIFICATION

(11) **EP 3 311 784 B1**
(45) Date of publication and mention of the grant of the patent: **06.02.2019**
(21) Application number: 17194881.3
(22) Date of filing: 05.10.2017
(51) Int. Cl.: A61F 13/15, B65G 41/00, B65G 47/00, B65G 57/00

(54) **AN APPARATUS AND METHOD FOR FORMING GROUPS OF PRODUCTS**
VORRICHTUNG UND VERFAHREN ZUR AUSBILDUNG VON GRUPPEN VON PRODUKTEN
APPAREIL ET PROCÉDÉ DE FORMATION DE GROUPES DE PRODUITS

(30) Priority: 20.10.2016 IT 201600105805
(43) Date of publication of application: 25.04.2018
(73) Proprietor: Fameccanica.Data S.p.A., 65129 Pescara (IT)
(72) Inventor: SABLONE, Gabriele, 65125 Pescara (IT)
(74) Representative: Marchitelli, Mauro

(56) References cited:
- WO-A1-2014/072951
- DE-A1-102011 107 290

## Description

### Field of the invention

The present invention relates to packaging of products and relates to an apparatus and a method for forming groups of products starting from a continuous flow of products advancing along a machine direction. The invention specifically relates to forming groups of products that must be compacted prior to packaging.

In the following description, reference will be made to disposable absorbent sanitary articles without removing genericity from the discussion.

### Description of the prior art

Disposable absorbent sanitary articles, such as sanitary towels, and diapers for babies or the like, contain a considerable amount of air. Packaging of these products comprises a step of forming groups of products and a compacting step in which the groups of products are compressed prior to inserting them into packaging wrap. The compacting step of the groups of products is required to form packages with reduced dimensions.

In the prior art, there are apparatuses for forming groups of products comprising an inlet line in which the products advance along a first direction, and a conveyor having a plurality of seats that receive respective products from the inlet line. The conveyor moves the products along a direction transverse to the inlet direction and an extractor device extracts successive groups of products from the conveyor. The groups of products thus formed are sent to a compacting device, which compresses the groups of products in a transverse direction, upstream of a packaging station in which the groups of compacted products are inserted within respective wrappings.

The current trend of the market requires packages containing an increasingly lower number of products. This market trend limits the potentiality of production lines of absorbent sanitary articles. For example, a machine for producing sanitary towels is typically able to produce up to 1800 products per minute. A packaging machine is normally able to produce one hundred packs per minute. Currently, the sanitary towel packages most in demand on the market are those that contain ten items. Therefore, if packages of ten items per package are produced, the maximum speed of the system is limited to 1000 products per minute, which is a severe limitation of the machine speed that is able to produce up to 1800 products per minute.

Currently, this problem is resolved by installing two packing machines in parallel downstream from the apparatus for forming groups of products. This solution involves an increase in the cost due to doubling of the number of packaging machines, a limitation in the maximum number of products per package due to the distance between the extractor devices (except for very complex format changes) and an increase in the productive surfaces occupied.

### Object and summary of the invention

The present invention aims to provide an apparatus and a method for forming groups of products that overcome the aforesaid drawbacks.

According to the present invention, this object is achieved by an apparatus and a method having the characteristics forming the subject of claims 1 and 5.

The claims form an integral part of the disclosure provided here in relation to the invention.

### Brief description of the drawings

The present invention will now be described in detail with reference to the attached drawings, given purely by way of non-limiting example, wherein:

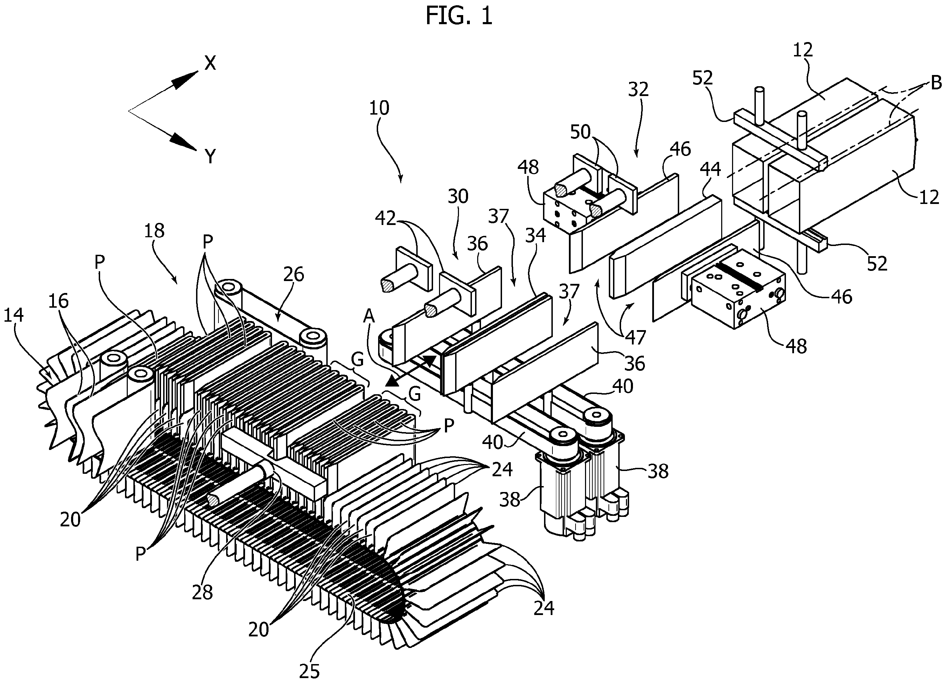

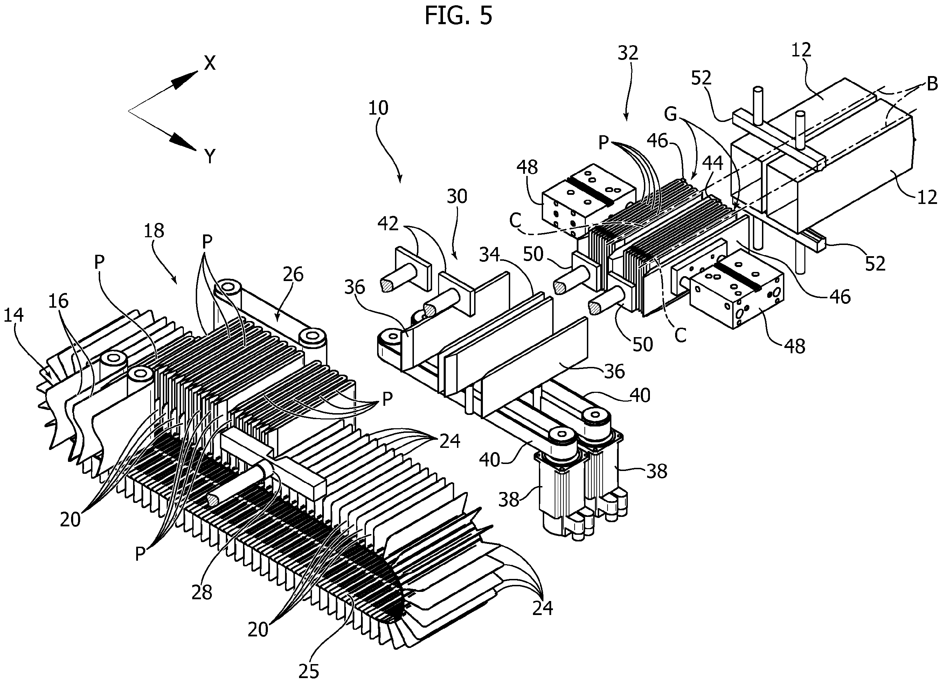

- Figures 1-8 are schematic perspective views illustrating the operating sequence of an apparatus according to the present invention.

### Detailed description

With reference to the Figures, numeral 10 indicates an apparatus for forming groups of products G intended to be packaged in respective wrappings 12.

The apparatus 10 comprises an inlet line 14 which advances individual products P aligned with each other along a first direction X. In the illustrated example, the inlet line comprises two conveyor belts having vertically oriented portions 16 facing each other, configured to hold and advance the individual products P in the direction X. In the example shown, the products P are disposable absorbent sanitary articles with a flat shape, which are advanced in the inlet line 14 oriented vertically, i.e. with the general planes of the individual products P vertically oriented.

The apparatus 10 comprises a conveyor 18 having a plurality of seats 20 for receiving respective products P. The seats 20 are formed by septa 24 extending orthogonally to the main surface of an endless conveyor belt 25. The conveyor belt 25 is driven in a second direction Y perpendicular to the first direction X. The septa 24 are spaced apart from each other by a constant pitch in the direction Y, and a seat 20 is defined for a respective product P between each pair of adjacent septa 24. The products P are inserted into the respective seats 20 with a movement in the direction X. The movement of the conveyor 18 in the direction Y is coordinated with the advancing speed of the products along the inlet line 14, so that each product P leaving the line 14 finds a respective free seat 20. The conveyor 18 can be provided with a vertically oriented containing belt 26, which forms a vertical surface against which the ends of the products P -protruding from the seats 20 on the opposite side to the insertion side- are supported.

The apparatus 10 comprises an extractor element 28, which is cyclically movable along an extracting direction indicated by the double arrow A to extract, at each run, two groups of products G from each of the seats 20 of the conveyor 18. The direction A, along which the extractor element 28 is movable, is parallel to the first direction X. The extractor element 28 rests against the front sides of the products P that protrude beyond the septa 24. Each group of products G is formed by a predetermined number of products P, corresponding to the width of the extractor element 28. Preferably, the groups of products G are separated from each other on the conveyor 18 by an empty seat 20. This is obtained in the step of inserting the products P into the seats 20, by advancing the conveyor 18 by a pitch equal to the width of the seats 20, without advancing a new product P after having loaded a number of products P, corresponding to the number of products forming each group G, onto the conveyor 18.

The apparatus 10 comprises at least one compacting device for compacting the groups of products G in the transverse direction Y. In the example illustrated, the apparatus 10 comprises a first compacting device 30 and a second compacting device 32 arranged in series with each other in the direction A. However, the presence of two compacting devices in series with each other is not imperative.

The first compacting device 30 comprises a fixed central wall 34 and two side walls 36 that are cyclically movable in the transverse direction Y towards the central wall 34 between an open position and a closed position. Two first compacting zones 37 are defined between the central wall 34 and the side walls 36. The walls 34, 36 are vertically oriented and actuated by respective motors 38. In the illustrated example, the side walls 36 are connected to the motors 38 by means of respective belt transmissions 40. The first compacting device 30 comprises a pair of pushers 42 configured to move the groups of products G in the direction A after the compacting step of the first compacting device 30.

The second compacting device 32 (optional) comprises a fixed central wall 44 aligned in the direction A with the fixed central wall 34 of the first compactor device 30 and two side walls 46, which are cyclically movable in the transverse direction Y towards the fixed central wall 44 between an open position and a closed position. Two second compacting zones 47 are defined between the central wall 44 and the side walls 46. The side walls 46 are driven by means of respective actuators 48. The second compacting device 32 comprises two pusher elements 50 configured to move the groups of products G in the direction A after the compacting step of the second compacting device 32.

Downstream of the second compacting device 32, a packaging machine is provided for forming tubular wrappings 12, which are sealed by means of a welding device 52. Any type of packaging machine known in the art for packaging absorbent sanitary articles can be used to form the tubular wrappings 12.

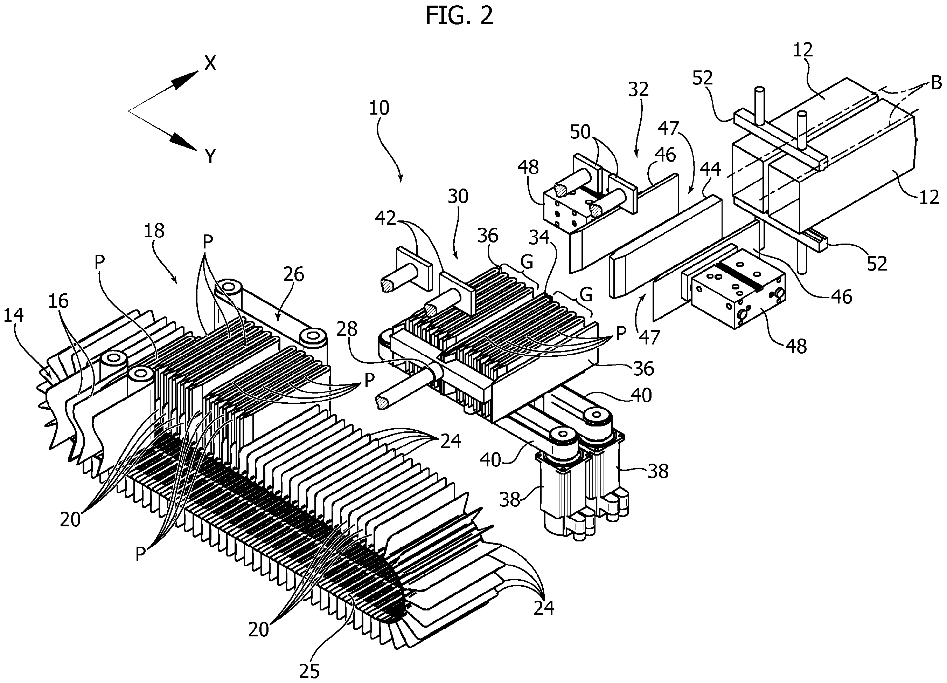

The operation of the apparatus according to the present invention is as follows. In the configuration illustrated in Figure 1, the first and the second compacting devices 30, 32 are in an open position and two tubular wrappings 12, with an open end, are arranged with the open end facing the outlet section of the second compacting device 32. The pusher devices 42, 50 of the compacting devices 30, 32 are in inoperative positions. On the conveyor 18, successive groups of products G are formed, separated from each other by an empty seat 20. When the seat 20 between two groups of products G is aligned in the direction A with the fixed central wall 34 of the first compacting device 30, the extractor device 28 is activated so as to push two groups of products G in the direction A towards the first compacting device 30, as shown in Figure 2.

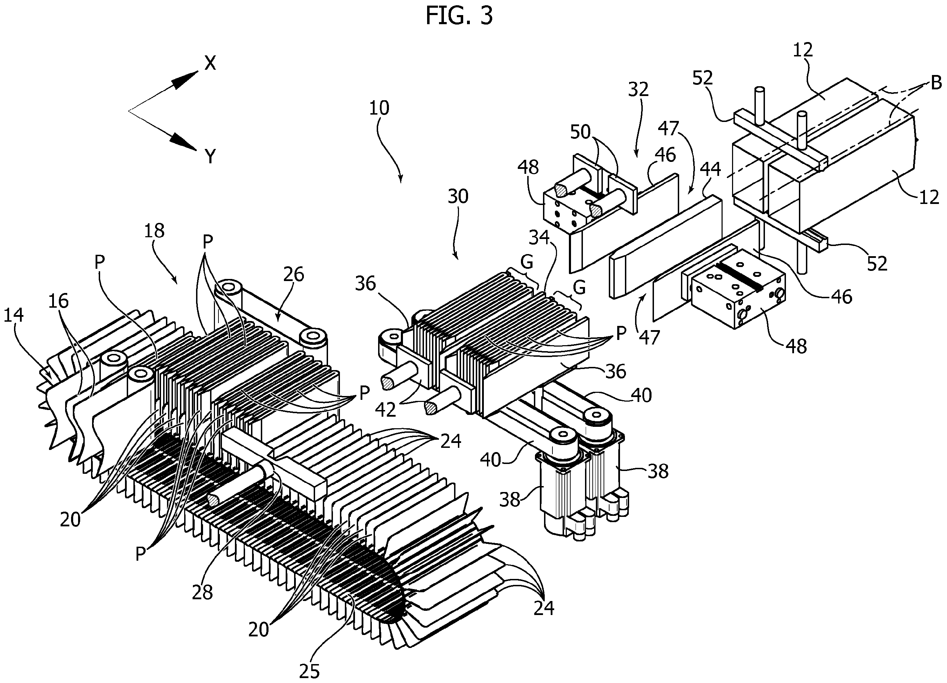

At this point, as illustrated in Figure 3, the two side walls 36 of the first compacting device 30 are moved towards the central wall 34 in the transverse direction Y, thereby effecting a first compacting of the two groups of products G. Then, the two pushers 42 are positioned behind the two groups of products G located in the first compacting device 30.

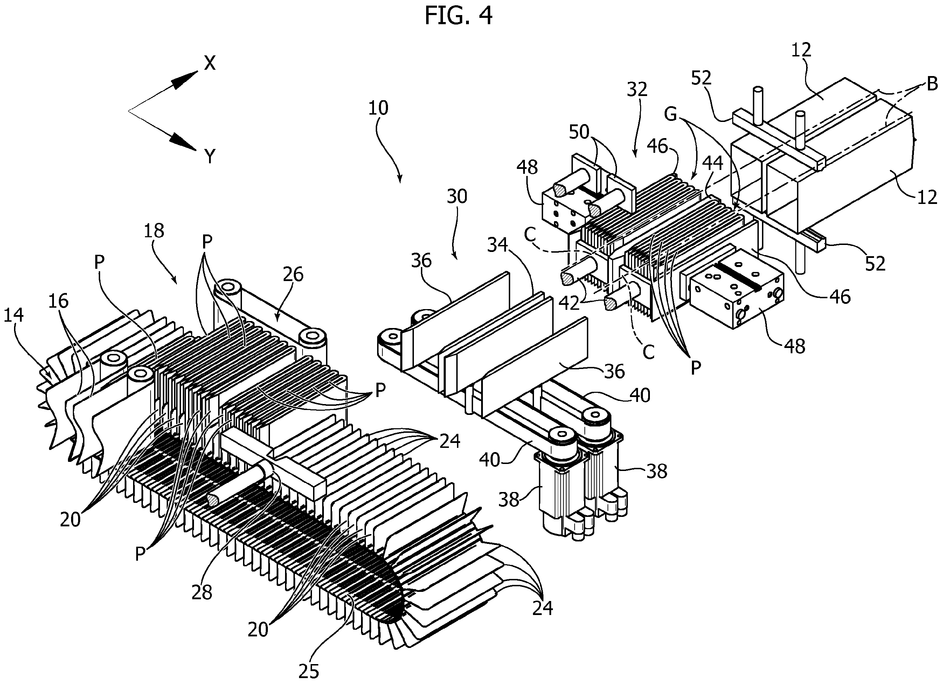

Then, the first pushers 42 are moved in the direction A to transfer the two groups of products G from the first compacting device 30 to the second compacting device 32, as illustrated in Figure 4.

The second compacting device 32 carries out a second compacting of the groups of products G, with a greater compression force than the compression force of the first compacting device 30. With reference to Figure 4, at the end of the second compacting step, the longitudinal axes C of the two groups of products G are aligned with the longitudinal axes B of the respective wrappings 12.

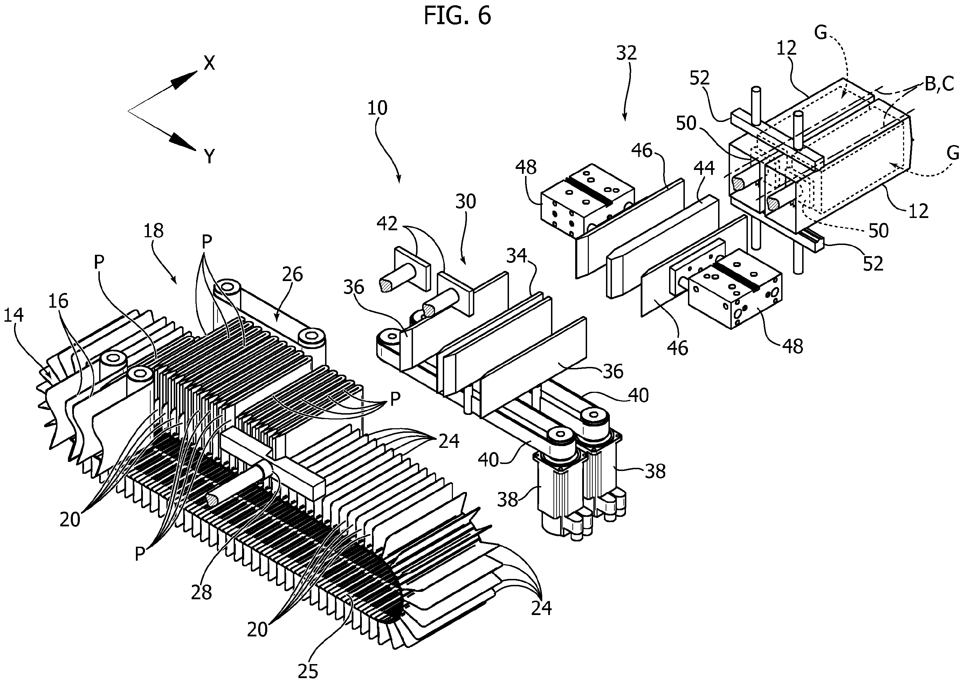

After the second compacting step, the second pushers 50 transfer the two groups of products G into the respective tubular wrappings 12 as shown in Figure 6.

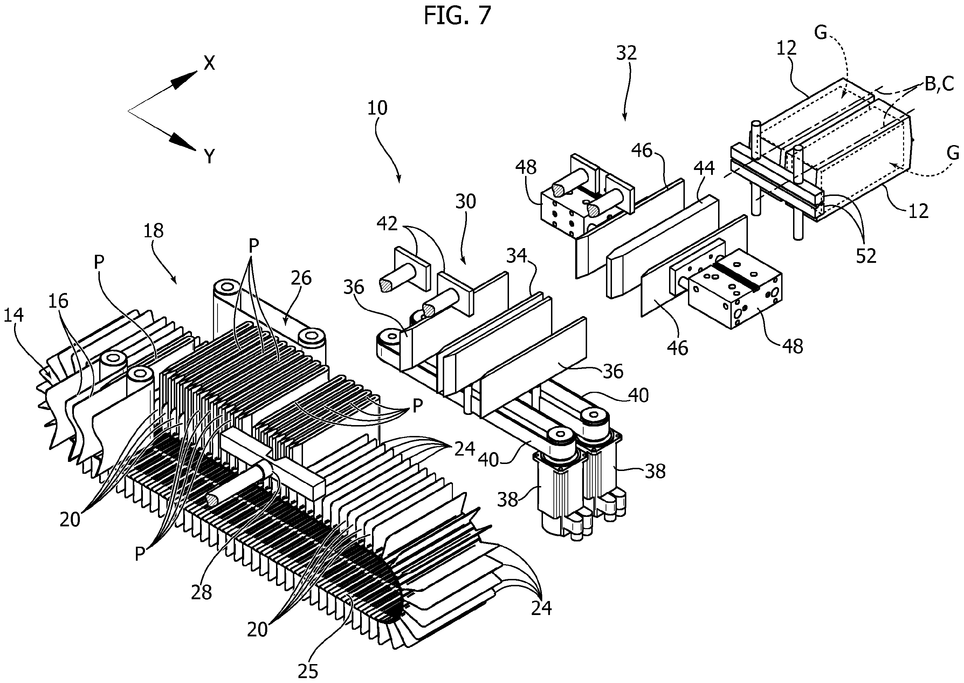

The second pushers 50 are then extracted from the wrappings 12 and the welding device 52 closes and creates a weld that closes the two open ends of the tubular wrappings 12, as illustrated in Figure 7.

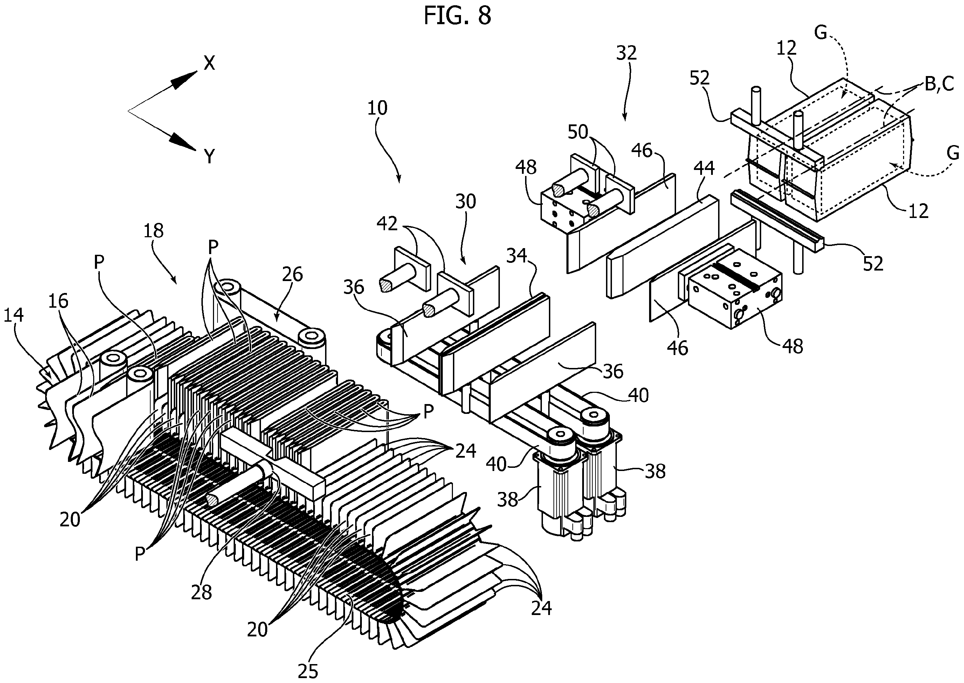

At this point, as illustrated in Figure 8, the sealed packages are sent to a secondary packaging station and the apparatus is ready for a new cycle.

A known type of packaging machine configured to form and seal 100 packages per minute by simultaneously forming two side-by-side wrappings would have a production capacity of 200 packages per minute. Considering, for example, packages made up of ten products, a single packaging machine can, therefore, package up to 2000 products per minute, and can process the entire production of a machine with a production capacity of 1800 products per minute.

The compacting devices 30, 32 are capable of compacting two groups of products G with the distance between the longitudinal axis C of the two groups of products G equal to the distance between the longitudinal axes B of the wrappings 12 produced by a single packaging machine. In this way, the costs associated with the use of two separate packaging machines are avoided, and the productive space occupied is reduced compared to solutions employing two packaging machines.

Of course, without prejudice to the principle of the invention, the details of construction and the embodiments can be widely varied with respect to those described and illustrated, without thereby departing from the scope of the invention as defined by the claims that follow.

## Claims

1. An apparatus for forming groups of products, comprising:
- an inlet line (14) arranged to advance individual products (P) aligned along a first direction (X),
- a conveyor (18) having a plurality of seats (20) for receiving respective products (P) from said inlet line (14) and movable along a second direction (Y) orthogonal to said first direction (X),
- an extractor element (28) movable cyclically along an extraction direction (A) parallel to said first direction (X) and arranged to extract a first and a second group of products (G) from said conveyor (18) at each cycle, and
- at least one compacting device (30, 32) comprising a fixed central wall (34, 44) and two side walls (36, 46) movable cyclically with respect to the central wall (34, 44) between an open position and a closed position, wherein said side walls (36, 46) and said fixed wall (34, 44) define two compacting zones (37, 47) for receiving respective groups of products (G) from said conveyor (18), wherein said side walls (36, 46) compact said groups of products (G) against the central wall (34, 44) during movement from the open position to the closed position.

2. An apparatus according to claim 1, wherein said at least one compacting device (30, 32) comprises a pair of pusher elements (42, 50) configured to push the groups of compacted products (G) along said extraction direction (A).

3. An apparatus according to claim 1 or claim 2, comprising a first and a second compacting device (30, 32) arranged in series with each other along said extraction direction (A).

4. An apparatus according to any of the preceding claims, comprising a packaging machine configured to form two side-by-side tubular wrappings (12) having respective longitudinal axes (B) aligned with the longitudinal axes (C) of the groups of compacted products (G) coming from said at least one compacting device (30, 32).

5. A method for forming groups of products, comprising:
- advancing individual products (P) aligned along a first direction (X),
- arranging said products (P) within respective seats (20) of a conveyor (18) and moving said products (P) along a second direction (Y) orthogonal to said first direction (X),
- cyclically extracting a first and a second group of products (G) from said conveyor (18), along an extraction direction (A) parallel to the said first direction (X), and
- compacting said first and second group of products (G) in said second direction (Y) between a fixed central wall (34, 44) and two side walls (36, 46) movable towards said fixed central wall (34, 44).

6. A method according to claim 5, comprising the step of leaving an empty seat (20) between each pair of adjacent groups of products (G) on said conveyor (18) and positioning said empty seat (20) aligned with said central fixed wall (34, 44) during said extraction step.

7. A method according to claim 5, comprising the step of advancing said groups of compacted products (G) along said extraction direction (A) by means of respective pushers (42, 50).

8. A method according to any one of claims 5-7, comprising the step of performing a first and a second compacting of said groups of products (G) in series along said extraction direction (A).

9. A method according to any one of claims 5-8, comprising the step of forming pairs of tubular wrappings (12) arranged with their respective longitudinal axes (B) aligned with the longitudinal axes (C) of said groups of compacted products (G).

## Patentansprüche

1. Vorrichtung zum Bilden von Gruppen von Produkten, umfassend:
- eine Zuführung (14), die dafür eingerichtet ist, entlang einer ersten Richtung (X) ausgerichtete einzelne Produkte (P) zu transportieren,
- eine Fördereinrichtung (18), die eine Vielzahl von Aufnahmen (20) zum Empfangen jeweiliger Produkte (P) von der Zuführung (14) aufweist und entlang einer orthogonal zu der ersten Richtung (X) verlaufenden zweiten Richtung (Y) bewegbar ist,
- ein Auswerfelement (28), das zyklisch entlang einer parallel zu der ersten Richtung (X) verlaufenden Auswerfrichtung (A) bewegbar ist und dafür eingerichtet ist, in jedem Zyklus eine erste und eine zweite Gruppe (G) von Produkten aus der Fördereinrichtung (18) auszuwerfen, und
- mindestens eine Verdichtungseinrichtung (30, 32), die eine feststehende Mittelwand (34, 44) und zwei Seitenwände (36, 46) umfasst, die bezüglich der Mittelwand (34, 44) zyklisch zwischen einer offenen Position und einer geschlossenen Position bewegbar sind, wobei die Seitenwände (34, 46) und die feststehende Wand (34, 44) zwei Verdichtungszonen (37, 47) zum Empfangen jeweiliger Gruppen (G) von Produkten von der Fördereinrichtung (18) definieren, wobei die Seitenwände (36, 46) während einer Bewegung aus der offenen in die geschlossene Position die Gruppen (G) von Produkten gegen die Mittelwand (34, 44) verdichten.

2. Vorrichtung nach Anspruch 1, wobei die mindestens eine Verdichtungseinrichtung (30, 32) ein Paar Schiebeelemente (42, 50) umfasst, die dafür ausgelegt sind, die Gruppen (G) von verdichteten Produkten entlang der Auswerfrichtung (A) zu schieben.

3. Vorrichtung nach Anspruch 1 oder Anspruch 2, umfassend eine erste und eine zweite Verdichtungseinrichtung (30, 32), die entlang der Auswerfrichtung (A) in Reihe geschaltet sind.

4. Vorrichtung nach einem der vorhergehenden Ansprüche, umfassend eine Verpackungsmaschine, die dafür ausgelegt ist, zwei nebeneinanderliegende röhrenförmige Umhüllungen (12) zu bilden, die jeweilige Längsachsen (B) aufweisen, die mit den Längsachsen (C) der von der mindestens einen Verdichtungseinrichtung (30, 32) kommenden Gruppen (G) von verdichteten Produkten ausgerichtet sind.

5. Verfahren zum Bilden von Gruppen von Produkten, umfassend:
- Transportieren von entlang einer ersten Richtung (X) ausgerichteten einzelnen Produkten (P),
- Anordnen der Produkte (P) in jeweiligen Aufnahmen (20) einer Fördereinrichtung (18) und Bewegen der Produkte (P) entlang einer orthogonal zu der ersten Richtung (X) verlaufenden zweiten Richtung (Y),
- zyklisches Auswerfen einer ersten und einer zweiten Gruppe (G) von Produkten aus der Fördereinrichtung (18) entlang einer parallel zu der ersten Richtung (X) verlaufenden Auswerfrichtung (A) und
- Verdichten der ersten und zweiten Gruppe (G) von Produkten in der zweiten Richtung (Y) zwischen einer feststehenden Mittelwand (34, 44) und zwei in Richtung der feststehenden Mittelwand (34, 44) bewegbaren Seitenwänden (36, 46).

6. Verfahren nach Anspruch 5, umfassend den Schritt des Leerlassens einer Aufnahme (20) zwischen jedem Paar benachbarter Gruppen (G) von Produkten auf der Fördereinrichtung (18) und des Positionierens der leeren Aufnahme (20) in Ausrichtung mit der feststehenden Mittelwand (34, 44) während des Auswerfschritts.

7. Verfahren nach Anspruch 5, umfassend den Schritt des Transportierens der Gruppen (G) von verdichteten Produkten entlang der Auswerfrichtung (A) mittels jeweiliger Schieber (42, 50).

8. Verfahren nach einem der Ansprüche 5 bis 7, umfassend den Schritt des Durchführens eines entlang der Auswerfrichtung (A) in Reihe erfolgenden ersten und zweiten Verdichtens der Gruppen (G) von Produkten.

9. Verfahren nach einem der Ansprüche 5 bis 8, umfassend den Schritt des Bildens von Paaren aus röhrenförmigen Umhüllungen (12), deren jeweilige Längsachsen (B) in Ausrichtung mit den Längsachsen (C) der Gruppen (G) von verdichteten Produkten angeordnet sind.

## Revendications

1. Appareil de formation de groupes de produits, comprenant :
- une ligne d'entrée (14) agencée pour faire avancer des produits individuels (P) alignés le long d'une première direction (X),
- un convoyeur (18) ayant une pluralité de sièges (20) pour recevoir des produits respectifs (P) de ladite ligne d'entrée (14) et pouvant être déplacé le long d'une deuxième direction (Y) orthogonale à ladite première direction (X),
- un élément extracteur (28) pouvant être déplacé de manière cyclique le long d'une direction d'extraction (A) parallèle à ladite première direction (X) et agencé pour extraire des premier et deuxième groupes de produits (G) dudit convoyeur (18) à chaque cycle, et
- au moins un dispositif de compactage (30, 32) comprenant une paroi centrale fixe (34, 44) et deux parois latérales (36, 46) pouvant être déplacées de manière cyclique par rapport à la paroi centrale (34, 44) entre une position ouverte et une position fermée, où lesdites parois latérales (36, 46) et ladite paroi fixe (34, 44) définissent deux zones de compactage (37, 47) pour recevoir des groupes respectifs de produits (G) dudit convoyeur (18), où lesdites parois latérales (36 , 46) compactent lesdits groupes de produits (G) contre la paroi centrale (34, 44) pendant le déplacement de la position ouverte à la position fermée.

2. Appareil selon la revendication 1, dans lequel ledit au moins un dispositif de compactage (30, 32) comprend une paire d'éléments poussoirs (42, 50) configurés pour pousser les groupes de produits compactés (G) le long de ladite direction d'extraction (A).

3. Appareil selon la revendication 1 ou 2, comprenant des premier et deuxième dispositifs de compactage (30, 32) agencés en série l'un avec l'autre le long de ladite direction d'extraction (A).

4. Appareil selon l'une des revendications précédentes, comprenant une machine d'emballage configurée pour former deux enveloppements tubulaires côte à côte (12) ayant des axes longitudinaux respectifs (B) alignés avec les axes longitudinaux (C) des groupes de produits compactés (G) provenant dudit au moins un dispositif de compactage (30, 32).

5. Procédé de formation de groupes de produits comprenant le fait :
- de faire avancer des produits individuels (P) alignés le long d'une première direction (X),
- d'agencer lesdits produits (P) dans des sièges respectifs (20) d'un convoyeur (18) et de déplacer lesdits produits (P) le long d'une deuxième direction (Y) orthogonale à ladite première direction (X)
- d'extraire de manière cyclique des premier et deuxième groupes de produits (G) dudit convoyeur (18), le long d'une direction d'extraction (A) parallèle à ladite première direction (X), et
- de compacter lesdits premier et deuxième groupes de produits (G) dans ladite deuxième direction (Y) entre une paroi centrale fixe (34, 44) et deux parois latérales (36, 46) pouvant être déplacées vers ladite paroi centrale fixe (34, 44).

6. Procédé selon la revendication 5, comprenant l'étape consistant à laisser un siège vide (20) entre chaque paire de groupes adjacents de produits (G) sur ledit convoyeur (18) et à positionner ledit siège vide (20) aligné avec ladite paroi centrale fixe (34, 44) pendant ladite étape d'extraction.

7. Procédé selon la revendication 5, comprenant l'étape consistant à faire avancer lesdits groupes de produits compactés (G) le long de ladite direction d'extraction (A) au moyen de poussoirs respectifs (42, 50).

8. Procédé selon l'une quelconque des revendications 5 à 7, comprenant l'étape consistant à effectuer des premier et deuxième compactages desdits groupes de produits (G) en série le long de ladite direction d'extraction (A).

9. Procédé selon l'une quelconque des revendications 5 à 8, comprenant l'étape consistant à former des paires d'enveloppements tubulaires (12) agencées avec leurs axes longitudinaux respectifs (B) alignés avec les axes longitudinaux (C) desdits groupes de produits compactés (G).
